# EUROPEAN PATENT SPECIFICATION

(11) **EP 3 784 204 B1**
(45) Date of publication and mention of the grant of the patent: **13.12.2023**
(21) Application number: 19727285.9
(22) Date of filing: 26.04.2019
(51) Int. Cl.: A61K 8/34, A61K 8/36, A61K 8/37, A61K 8/81, A61K 9/00, A61K 47/10, A61K 47/12, A61K 47/14, A61K 47/22, A61K 47/26, A61K 47/32, A61Q 3/02

(54) **KIT OF PARTS FOR NAIL FUNGUS**
KIT FÜR NAGELPILZE
KIT POUR LE CHAMPIGNON DES ONGLES

(30) Priority: 27.04.2018 DK PA201870250
(43) Date of publication of application: 03.03.2021
(73) Proprietor: Unigroup ApS, 2800 Lyngby (DK)
(72) Inventor: LICHT, Flemming, 2800 Lyngby (DK)
(74) Representative: Holme Patent A/S
(86) International application number: PCT/DK2019/050128
(87) International publication number: WO 2019/206389

(56) References cited:
- EP-A2- 2 226 078
- WO-A1-99/53913
- WO-A1-2012/107565
- WO-A1-2017/062761
- WO-A2-2007/094999
- WO-A2-2011/053139
- DATABASE GNPD [Online] MINTEL; 2 September 2014 (2014-09-02), anonymous: "3 in 1 Yellow Nail Care", XP055605997, retrieved from www.gnpd.com Database accession no. 2632589
- BIANCA PIRACCINI ET AL: "Onychomycosis: A Review", JOURNAL OF FUNGI, vol. 1, no. 1, 27 March 2015 (2015-03-27), pages 30-43, XP055605472, DOI: 10.3390/jof1010030

## Description

The present invention relates to kit of parts comprising a base coat composition and a nail composition. Further, the invention concerns kit of parts for topical application, therapeutic uses and non-therapeutic uses. The invention further concerns kit of parts as well as a base coat for the treatment of nail fungus. In particular, the invention concerns kit of parts comprising a peel off base coat composition and a nail composition for the treatment of nail fungus.

### Technical Background

The patent document WO2018041319 (LICHT, F.) relates to a composition comprising ozonated oil. The invention further concerns a composition for the treatment of nail fungus. In particular, the invention concerns a composition comprising ozonated oil for the treatment of nail fungus.

The patent document US6740326 B1 (MEYER, H. et al.) relates to a water-free topical application composition for the treatment of nail diseases and nail care consisting essentially of one or more antimycotic substances; and one or more C1-C4-alkyl esters as carrier, wherein the carrier is a penetration promoting substance.

The patent document US2012247496 (TAYLOR, T.) relates to compositions and methods for forming a more durable, longer lasting and chip-resistant coating of nail polish upon a fingernail or toenail. The composition comprises a combination of artificial nail builders and photoinitiators, as well as other ingredients, which form a base coat composition. In use, the base coat composition is first applied directly upon the fingernail or toenail and thereafter cured by either UV or LED lighting.

The patent document US20150342871 (BUYUKTIMKIN, S. et al) relates to an anti-fungal pharmaceutical composition for topical application for the treatment of onychomycosis. The composition comprises an anti-fungal agent, a zwitterionic surfactant or charged derivative thereof; a carboxylic acid, a lower alcohol, water and a thickener.

The patent document US6143794 (CHAUDHURI, B. et al) relates to a topical formulation useful for treating a nail fungal disease and a method of treating fungal disease in nails. The composition comprises an antifungal compound, at least one pharmaceutically acceptable excipient, gelling agent, adhesion-promoting agent, film-forming agent, surfactant, keratolytic agent and water, wherein the film-forming agent is polyvinyl alcohol.

The article "Transonychial water loss in healthy and diseased nails" by Krönauer C et al. 2001 describes changes in transepidermal water loss (TEWL) and transonychial water loss (TOWL) from the skin and the nails in patients suffering from atopic eczema, psoriasis and onychomycosis compared to healthy individuals.

The article "A few aspects of transonychial water loss (TOWL): inter-individual, and intra-individual inter-finger, inter-hand and inter-day variabilities, and the influence of nail plate hydration, filing and varnish" by Murdan S. et al. 2008 describes a study that measures transonychial water loss (TOWL) in order to identify the extent of inter-individual, intra-individual inter-finger, inter-hand, and inter-day variabilities, and the influence of nail wetting, filing and varnishing on TOWL, with a view to determine parameters for the measurement of TOWL and its possible applications.

### Summary of the invention

The present invention concerns a kit of parts comprising a base coat composition and a nail composition, wherein said base coat composition comprises:
A. Water,
B. An alcohol, preferably ethanol, and
C. Polyvinyl alcohol,
and wherein said nail composition comprises:
a. Lactic acid, and/or
b. At least one C1-C4 alkyl ester of lactic acid, malic acid, tartaric acid, citric acid, or a mixture of any of these.

Nail fungus is often associated with discoloration of the nail. Many people, especially women, prefer to have nicely looking and/or manicured nails. The present invention may provide a possibility for wearing nail polish and/or having manicured nails simultaneously with daily treatments of the nail for nail fungus without harming, degrading and/or destroying the nail.

When applying a composition to the nail, the treated person will most often have to wait for the composition to penetrate the nail. Penetration through the nail is important for the composition to dissipate in the nail and interact efficiently with any toenail and/or fingernail fungus. A nail composition of the invention may provide faster penetration from the nail surface through the nail. This faster penetration results in a faster removal from the nail surface, so the base coat composition and subsequent nail polish can be applied immediately after application of the nail composition.

A kit of parts of the present invention may provide a base coat composition acting as a protective adhesive colloid film with high oxygen barrier properties, hereby facilitating hydrolysis of components comprised in the nail composition, which hydrolysis results in a pH lowering effect within the nail.

A kit of parts of the present invention may provide a base coat composition acting as a protective adhesive colloid film with high oxygen barrier properties, hereby facilitating a moist environment within the nail, which moist environment may provide a pH lowering effect within the nail.

A kit of parts of the present invention may provide a hydrolysis of components comprised in said nail composition inside the nail after application of the nail composition on the nail, hereby lowering the pH inside the nail on a continued basis over time.

Nail fungus is often associated with discoloration of the nail. The present invention may provide a whitening effect which counteracts such discoloration.

A kit of parts of the present invention may provide a nail composition with improved penetration into a nail, and may facilitate complete penetration. This may provide better interaction between the composition and any toenail and/or fingernail fungus.

The treatment of nail fungus is often associated with a very long treatment time. A kit of parts of the invention may provide a faster effect, meaning that a shorter treatment time will be necessary.

A person with slowly growing nails may have an increased risk of having nail fungus. Nails that grow slowly may take longer time to recover from nail fungus. A kit of parts of the invention may accelerate the growth of the nail, leading to prevention, prophylaxis and/or faster curation from nail fungus.

A person with thickened nails may have an increased risk of having nail fungus and thickened nails take longer time to recover from nail fungus. A kit of parts of the invention may help the nail regain normal thickness faster, leading to prevention, prophylaxis and/or faster curation from nail fungus.

Fungal tests are used to help detect and diagnose a fungal infection, to help guide treatment, and/or to monitor the effectiveness of treatment. A negative test after a person has been treated for a fungal infection means that the therapy has been successful. A kit of parts of the invention may help reach a negative fungal test faster, ensuring that the source of the nail fungus is efficiently removed.

Ethyl lactate may act as a skin or nail penetration enhancer. A nail composition of the invention may provide a faster saturation of the nail, ensuring that the entire nail is saturated in a complete and fast way. Full saturation of the entire nail, including the nail bed and nail root, is important to protect the entire nail against nail fungus.

Nail fungus is a contagious disease, allowing spread within the nail, to other nails and even to the surrounding skin. Previous treatments for nail fungus only seek to cure the nail fungus. A composition of the invention may be capable of filling out all or substantially all cavities in the nail and/or of the surface of the nail, thereby preventing the fungi from dissipating in the nail and/or to the skin; in particular this will result in better protection of the nail and the skin from the fungi, decrease the probability of spread and/or decrease the probability of relapse.

A kit of parts of the invention may allow simultaneous treatment of the nail and the skin. Previous treatments focus on treating either one or the other.

A number of different factors have been reported to influence TOWL. It could be speculated that disrupted TOWL is a problem associated with nail conditions, such as nail fungus. A kit of parts of the invention may normalize TOWL in the nail and/or in the skin.

TOWL has been reported to be significantly lower in nails affected by atopic eczema and fungal infections, compared to healthy nails. A kit of parts of the present invention may increase TOWL in the nail and/or in the skin to levels associated with healthy nails.

According to an embodiment the present invention may be suitable for the treatment of atopic eczema.

According to an aspect, the present invention concerns a kit of parts comprising a base coat composition and a nail composition for use in a treatment.

According to an aspect, the present invention concerns a kit of parts comprising a base coat composition and a nail composition for a non-therapeutic use.

According to another aspect a method of treating nail fungus, comprising use of a kit of parts comprising a base coat composition and a nail composition is disclosed.

Usually, the use of nail polish or lacquer is discouraged, due to the risk of creating a humid environment under the nail polish or lacquer. However, the use of a base coat of the present invention hydrates and establishes a low pH under the coat, in which fungus and/or bacteria are inhibited.

It may be speculated that as lactic acid is hygroscopic, and attracts water, such as from the base coat, it may be providing synergy to the hydrolysis process. A kit of parts of the present invention may inhibit fungus and/or bacteria growth in the nail and/or the skin due to a lowering of the pH under the coat.

A method for decreasing TOWL is disclosed, wherein said method comprises daily application of a kit of parts comprising a base coat composition and a nail composition to the nails of a subject, and wherein said method comprises the following steps:
I. Removal of any nail polish from the nail using said base coat composition, providing a clean nail surface,
II. Subsequent application of said nail composition on the clean nail surface,
III. Subsequent application of a new layer of said base coat composition, and
IV. Subsequent application of a new layer of nail polish on the nail.

A method for reducing wash-out of a nail composition applied to the nails of a subject is disclosed, wherein said wash-out is due to hand wash, showering and/or increase in relative humidity of the surrounding environment, wherein said method comprises daily application of a kit of parts comprising a base coat composition and a nail composition to the nails of a subject, and wherein said method comprises the following steps:
I. Removal of any nail polish from the nail using said base coat composition, providing a clean nail surface,
II. Subsequent application of said nail composition on the clean nail surface,
III. Subsequent application of a new layer of said base coat composition, and
IV. Subsequent application of a new layer of nail polish on the nail.

A method for preventing filing-induced TOWL is disclosed, wherein said method comprises daily application of a kit of parts comprising a base coat composition and a nail composition to the nails of a subject, and wherein said method comprises the following steps:
I. Removal of any nail polish from the nail using said base coat composition, providing a clean nail surface,
II. Subsequent application of said nail composition on the clean nail surface,
III. Subsequent application of a new layer of said base coat composition, and
IV. Subsequent application of a new layer of nail polish on the nail.

### Detailed Disclosure

According to different embodiments, the present invention concerns the kit of parts, compositions, methods and uses of the claims.

The nail plate is a hard, yet slightly elastic, translucent, convex structure. The nail plate mainly consists of the fibrous protein, keratin. The nail consists of three different layers: The dorsal (upper) layer, the intermediate layer, and the ventral (buttom) layer.

Within this disclosure, the term "peel off base coat" is used interchangeable with the term "peel-off base coat", and is understood to be a composition applied on the nails before application of nail polish. The peel off base coat may allow easy removable of nail polish from the nails by peeling off the base coat and the subsequently applied nail polish in one step.

According to an embodiment, the invention concerns a kit of parts, wherein said base coat composition is a peel off base coat.

According to an embodiment, the invention concerns a kit of parts, wherein said nail composition penetrates and/or dissipates in the dorsal layer of the nail, more preferred the dorsal layer and the intermediate layer of the nail, preferably the dorsal layer, the intermediate layer and the ventral layer of the nail.

Within this disclosure, the term "nail polish" is used interchangeable with similar terms, such as nail lacquer, lacquer and nail varnish.

According to an embodiment, the invention concerns a kit of parts, wherein said nail composition provides a fast penetration from the nail surface through the nail, wherein said fast penetration allows immediate subsequent application of said base coat composition on a dry nail surface. According to a preferred embodiment, this is possible without prior filing or scraping of the nail.

According to an embodiment, the invention concerns a kit of parts, wherein said fast penetration allows application of the base coat composition on a dry nail surface within less than 15 minutes, preferably less than 10 minutes, more preferred less than 5 minutes, preferably less than 4 minutes, more preferred less than 3 minutes, preferably less than 2 minutes, more preferred less than 1 minute, preferably less than 30 seconds.

According to an embodiment, the invention concerns a kit of parts, wherein said base coat composition dries immediately.

According to an embodiment, the invention concerns a kit of parts, wherein said base coat composition dries within less than 15 minutes, preferably less than 10 minutes, more preferred less than 5 minutes, preferably less than 4 minutes, more preferred less than 3 minutes, preferably less than 2 minutes, more preferred less than 1 minute, preferably less than 30 seconds.

Said kit of parts may have an treatment cycle within less than 15 minutes, preferably less than 10 minutes, more preferred less than 5 minutes, preferably less than 4 minutes, more preferred less than 3 minutes, preferably less than 2 minutes, more preferred less than 1 minute, preferably 40 seconds or less.

Within this disclosure, the term "treatment cycle" is understood to be application of said nail composition on a clean nail surface and subsequent application of said base coat composition.

Said base coat composition allows subsequent application of nail polish within less than 15 minutes, preferably less than 10 minutes, more preferred less than 5 minutes, preferably less than 4 minutes, more preferred less than 3 minutes, preferably less than 2 minutes, more preferred less than 1 minute, preferably less than 30 seconds.

Said base coat composition allows subsequent application of nail polish, and wherein said base coat composition allows simultaneous removal of said base coat composition and said nail polish from the nail without any need for applying and/or using additional components.

Said base coat composition allows simultaneous removal of said base coat composition and subsequently applied nail polish from the nail without any need for applying and/or using additional components.

Said base coat composition allows simultaneous removal of said base coat composition and subsequently applied nail polish from the nail without any need for applying acetone and/or nail polish remover on the nail.

Said base coat composition allows subsequent application and removal of nail polish on the nail on a daily basis, without harming and/or degrading and/or destroying the nail.

Said base coat composition may be free of polyvinyl acetate.

The total amount (w/w) of said water in the base coat composition is 0.05 - 99.9%, preferably 1 - 99%, more preferred 10 - 90%, preferably 20 - 85%, more preferred 25 - 85%, preferably 30 - 85%, more preferred 40 - 85%, preferably 50 - 85%, more preferred 50 - 80%, preferably 55 - 80%, more preferred 55 - 75%, preferably 55 - 70 %, more preferred 60 - 70%, preferably 65-70%, or the total amount (w/w) of said water in the base coat composition is at least 99.9%, preferably 99%, more preferred 90%, preferably 85%, more preferred 80%, preferably 75%, more preferred 70%, preferably 65%, more preferred 60%, preferably 55%, more preferred 50%, preferably 45%, more preferred 40%, preferably 35%, more preferred 30%, preferably 25%, more preferred 20%, preferably 15%, more preferred 10%, preferably 5%, more preferred at least 2%, or the total amount (w/w) of said water in the base coat composition is at most 99.9%, preferably 99%, more preferred 90%, preferably 85%, more preferred 80%, preferably 75%, more preferred 70%, preferably 65%, more preferred 60%, preferably 55%, more preferred 50%, preferably 45%, more preferred 40%, preferably 35%, more preferred 30%, preferably 25%, more preferred 20%, preferably 15%, more preferred 10%, preferably 5%, more preferred at most 2%.

The total amount (w/w) of said alcohol in the base coat composition is 1 - 90%, preferably 2 - 80%, more preferred 3 - 70%, preferably 4 - 60%, more preferred 5 - 50%, preferably 5 - 40%, more preferred 5 - 35%, preferably 5 - 30%, more preferred 6 - 30%, preferably 7 - 30%, more preferred 8 - 30%, preferably about 8 - 25%..

According to an embodiment, the invention concerns a kit of parts, wherein said alcohol in the base coat composition is a denatured alcohol.

According to an embodiment, the invention concerns a kit of parts, wherein said alcohol in the base coat composition is selected from the group consisting of a primary alcohol, a secondary alcohol, and a tertiary alcohol.

According to an embodiment, the invention concerns a kit of parts, wherein said alcohol in the base coat composition is selected from the group consisting of monohydric alcohols, polyhydric alcohols, unsaturated aliphatic alcohols, and alicyclic alcohols.

According to an embodiment, the invention concerns a kit of parts, wherein said alcohol in the base coat composition is selected from the group consisting of methanol, ethanol, propan-2-ol, butan-1-ol, pentan-1-ol, hexadecan-1-ol, ethane-1,2-diol, propane-1,2-diol, propane-1,2,3-triol, butane-1,2,3,4-tetraol, pentane-1,2,3,4,5-pentol, hexane-1,2,3,4,5,6-hexol, heptane-1,2,3,4,5,6,7-heptol, Prop-2-ene-1-ol, 3,7-Dimethylocta-2,6-dien-1-ol, Prop-2-yn-1-ol, cyclohexane-1,2,3,4,5,6-hexol, and 2-(2-propyl)-5-methyl-cyclohexane-1-ol.

According to an embodiment, the invention concerns a kit of parts, wherein said alcohol in the base coat composition is ethanol.

According to an embodiment, the invention concerns a kit of parts, wherein the total amount (w/w) of said polyvinyl alcohol in the base coat composition is 1 - 90%, preferably 2 - 80%, more preferred 3 - 70%, preferably 4 - 60%, more preferred 5 - 50%, preferably 10 - 40%, more preferred 10- 35%, preferably 15 - 35%, more preferred 15 - 30%, preferably about 15 - 20%.

According to an embodiment, the invention concerns a kit of parts, wherein said polyvinyl alcohol in the base coat composition is selected from the group consisting of fully saponified grades, partially saponified grades, defoamed grades, fine powder grades, low ash grades, and specialty grades, or a mixture of any of these.

According to an embodiment, the invention concerns a kit of parts, wherein said polyvinyl alcohol in the base coat composition is selected from the group consisting of partially hydrolyzed polyvinyl alcohols and fully hydrolyzed polyvinyl alcohol, or a mixture of any of these.

According to an embodiment, the invention concerns a kit of parts, wherein said polyvinyl alcohol in the base coat composition has a degree of hydrolysis between 65 - 100 mol%, preferably 65 - 99 mol%, more preferred 70 - 99 mol%, preferably 72 - 98 mol%, more preferred 74 - 97 mol%, preferably 76 - 96 mol%, more preferred 78 - 95 mol%, preferably 80 - 95 mol%, more preferred 82 - 94 mol%, preferably 84 - 93 mol%, more preferred 86 - 92 mol%.

According to an embodiment, the invention concerns a kit of parts, wherein said polyvinyl alcohol in the base coat composition has a pH between 4-8, preferably 4.5 - 8, more preferred 4.5 - 7.5, preferably 5 - 7.5, more preferred 5 - 7.

Said polyvinyl alcohol in the base coat composition has the chemical formula (C₂H₄O)ₓ, wherein x might be any integer between 1 and 100, preferably 1-90, more preferred 1-80, preferably 1-70, more preferred 1-60, preferably 1-50, more preferred 1-40, preferably 1-30, more preferred 1-20, preferably between 1-10.

Said base coat composition may further comprise a pigment.

According to an embodiment, the invention concerns a kit of parts, wherein said base coat composition further comprises a preservative, preferably phenoxyethanol, wherein the total amount (w/w) of said preservative is 0.01 - 15%, preferably 0.02 - 10%, more preferred 0.03 - 5%, preferably 0.04 - 2%, more preferred 0.05 - 1.5%, preferably 0.5- 1.5%, more preferred 0.6 - 1.4%, preferably about 1.0%.

According to an embodiment, the invention concerns a kit of parts, wherein said base coat composition further comprises a preservative, wherein said preservative is selected from the group consisting of parabens, formaldahyde releasers, isothiazolinones, phenoxyethanol, and organic acids.

According to an embodiment, the invention concerns a kit of parts, wherein said nail composition further comprises an oil, wherein said nail composition further comprises a total amount (w/w) of oil between 1 - 90%, preferably 2 - 80%, more preferred 3 - 70%, preferably 4 - 60%, more preferred 5 - 50%, preferably 6 - 40%, more preferred 7 - 30%, preferably 8 - 20%, more preferred 9 - 15%, preferably about 10%.

According to an embodiment, the invention concerns a kit of parts, wherein said at least one C1-C4 alkyl ester comprised in the nail composition is ethyl lactate.

According to an embodiment, the invention concerns a kit of parts, wherein the total amount (w/w) of C1-C4 alkyl ester, such as ethyl lactate, in the nail composition is 0.05 - 99.9%, preferably 1- 99.5%, more preferred 10 - 99%, preferably 20 - 98%, more preferred 30 - 97%, preferably 40 - 96%, more preferred 50 - 95%, preferably 60 - 94%, more preferred 70 - 93%, preferably 75 - 92%, more preferred 80 - 90%, preferably 80 - 91 %, more preferred 81 - 89%, preferably 82.5 - 87.5%.

According to an embodiment, the invention concerns a kit of parts, wherein the total amount (w/w) of C1-C4 alkyl ester, such as ethyl lactate, in said nail composition is less than 99.9%, preferably 90%, more preferred 80%, preferably 70%, more preferred 60%, preferably less than 50%.

According to an embodiment, the invention concerns a kit of parts, wherein the total amount (w/w) of lactic acid in the nail composition is 0.05 - 40%, preferably 0.1 - 25%, more preferred 0.2 - 15%, preferably 0.4 - 10%, more preferred 0.6 -5%, preferably 0.8 - 3%, more preferred 1 - 2.5%, preferably 1.2 -2%, more preferred about 1.5%.

According to an embodiment, the invention concerns a kit of parts, wherein said nail composition further comprises dimethyl isosorbide, wherein the total amount (w/w) of dimethyl isosorbide in said nail composition is 0.005-99.9%, preferably 1-99.5%, more preferred 5-90%, preferably 10-80%, more preferred 15-70%, preferably 20-60%, more preferred 25-50%, preferably 30-40%.

Said dimethyl isosorbide enhances penetration through the nail and/or the skin, improves spreadability and/or increases polarity of said nail composition.

According to an embodiment, the invention concerns a kit of parts, wherein said nail composition further comprises an oil, and wherein said oil in the nail composition is a plant oil, wherein said oil in the nail composition is selected among the group consisting of the plant oils of Flax (Linseed), Hemp (C. sativa), Evening primrose, Safflower, Chia, Kukui (candle nut), Perilla, Grape seed, Pumpkin seed, Sunflower, Walnut, Soybean, Corn, Wheat germ, Rape (Canola), Sesame, Cotton seed, Rice bran, Beechnut, Sweet almond, Olive, Avocado, and mixtures of these, or said oil in the nail composition is selected among the group consisting of the plant oils of Hemp Oil, Canola (Rape) Oil, Sunflower Oil, Olive Oil, Avocado Oil, and mixtures of these, or wherein said oil in the nail composition is selected among the group consisting of Flax (Linseed) Oil, Hemp Oil, Olive Oil, Sunflower Oil, and mixtures of these.

According to an embodiment, the invention concerns a kit of parts, wherein said base coat composition and/or said nail composition are selected among liquid, a paste, a solid, a cream, an ointment, a nail lacquer, a spray, an oil, a gel, a nail-polish, a serum, an applicator pen, a brush, and a cotton swab.

According to an embodiment, the invention concerns a kit of parts, wherein said base coat composition and/or said nail composition have a dosage form selected among a salve, an oil, a crème, a gel, a liquid, a nail-polish, a serum and a lacquer.

According to an embodiment, the invention concerns a kit of parts, wherein said base coat composition and/or said nail composition have an application form selected among an applicator pen, a brush, a spray and a cotton swab.

According to an embodiment, the invention concerns a kit of parts for the treatment of nail fungus, or of a nail for onychomycosis.

The kit of parts simultaneous treats nail fungus and prevents nail fungus to dissipate in the nail.

The kit of parts decreases the probability of spread and/or decreasing the probability of relapse.

Application of said base coat composition creates a moist environment within the nail, wherein said moist environment promotes a pH lowering effect within the nail, promotes hydrolysis of components comprised in said nail composition, and serves as a protective adhesive colloid layer on the nail. Said application of said base coat composition promotes hydrolysis of ethyl lactate comprised in said nail composition into lactic acid and ethanol, hereby establishing a pH lowering effect within the nail. Said lactic acid attracts and retains water within the nail.

So said kit of parts provides a simultaneously moist and pH lowering environment within the nail.

The oxygen barrier properties of a composition can be described by using the following three parameters:
1. Oxygen Transmission Rate (O2GTR): The quantity of oxygen gas passing through a unit area of the parallel surfaces of a plastic film per unit time under the conditions of test. The SI unit of transmission rate is the mol/(m²·s).
2. Oxygen Permeance (PO₂): The ratio of the O2GTR to the difference between the partial pressure of O₂ on the two sides of the film. The SI unit of permeance is the mol/(m²·s·Pa).
3. Oxygen Permeability Coefficient (P'O₂): The product of the permeance and the thickness of film. The SI unit of oxygen permeability is the mol/(m²·s·Pa).

Said base coat composition has high oxygen barrier properties, a high oxygen transmission rate and/or oxygen permeance and/or oxygen permeability coefficient, provides a faster saturation of the nail, is capable of filling out the majority of cavities in the nail, thereby preventing the fungi to dissipate in the nail and/or to the skin, allows simultaneous treatment of the nail and the skin, accelerates the growth of the nail, promotes that the nail regains normal thickness faster.

Said nail composition promotes that the nail regain normal thickness in less than 12 months, preferably less than 10 months, more preferred less than 6 months, preferably less than 5 months, more preferred less than 4 months, preferably less than 3 months, more preferred less than 2 months, preferably less than 1 month, more preferred less than 2 weeks.

Transonychial Water Loss (TOWL) is water loss from the body through the nail plate into the outside environment. TOWL is measured as amount of lost water per area per time and has the unit g/m²h. A wide range of TOWL values for healthy nails has been reported. Age and diseases, such as atopic eczema, psoriasis and onychomycosis, have been reported to change TOWL values. A lot of other factors, such as nail plate thickness, wetting the nails, filing and nail polish application influence the TOWL value too.

Transepidermal water loss (TEWL) is water loss from the body through the epidermis to the outside environment. TEWL is measured as amount of lost water per area per time and has the unit g/m²h. Skin damage or pathological conditions such as eczema can increase TEWL. TEWL is also affected by other factors such as humidity, temperature, seasons and hydration level of the skin.

Said kit of parts and said nail composition reduces and/or normalizes the TEWL/TOWL ratio in the nail. Said normalization is obtained by increasing the TEWL/TOWL ratio in the nail.

Said nail composition and said kit of parts reduces and/or normalizes TOWL from the nail to a TOWL value between 5-100, preferably 10-90, more preferred 15-85, preferably 20-80, more preferred 25-75, preferably 30-70, more preferred 35-65, preferably 40-60, more preferred 45-55, in the nail of persons suffering from fungal nail infections, such as onychomycosis.

Said nail composition provides a faster effect, resulting in a shorter treatment time, wherein said nail composition provides a treatment time of less than 24 months, preferably less than 18 months, more preferred less than 16 months, preferably less than 12 months, more preferred less than 6 months, preferably less than 5 months, more preferred less than 4 months, preferably less than 3 months, more preferred less than 2 months, preferably less than 1 month, more preferred less than 2 weeks.

According to the invention said nail composition allows a negative fungal test to be reached in less than 24 months, preferably less than 18 months, more preferred less than 16 months, preferably less than 12 months, more preferred less than 6 months, preferably less than 5 months, more preferred less than 4 months, preferably less than 3 months, more preferred less than 2 months, preferably less than 1 month.

In the kit of parts said base coat composition and/or said nail composition provide improved storage stability, wherein said base coat composition and/or said nail composition can be stored without color change 1 month, preferably 2 months, more preferred 4 months, preferably 8 months, more preferred 12 months, preferably 18 months, more preferred 24 months, preferably 30 months, more preferred 36 months, preferably 48 months, more preferred 60 months.

Said base coat composition and/or said nail composition have a durability of 1 month, preferably 2 months, more preferred 4 months, preferably 8 months, more preferred 12 months, preferably 18 months, more preferred 24 months, preferably 30 months, more preferred 36 months, preferably 48 months, more preferred 60 months.

According to an embodiment, the invention concerns a kit of parts wherein said base coat composition and said nail composition is mixed together prior to application on the nail.

According to an embodiment, the invention concerns a use of the kit of parts for the treatment of nail fungus or of onychomycosis in the nail. Use of the kit of parts prevents or counteracts discolored or thick nails, or alleviates discomfort associated with discolored or thick nails.

According to an embodiment, the invention concerns a base coat composition for use in a treatment comprising applying a nail composition.

According to an embodiment, the invention concerns a nail composition for use in a treatment comprising applying a base coat composition.

According to an embodiment, the invention concerns a base coat composition and a nail coat composition for use in a topical treatment of a nail for nail fungus, and/or for use in treatment of a nail for onychomycosis.

A method for decreasing TOWL is disclosed, wherein said method comprises daily application of a kit of parts comprising a base coat composition and a nail composition to the nails of a subject, and wherein said method comprises the following steps:
I. Removal of any nail polish from the nail using said base coat composition, providing a clean nail surface,
II. Subsequent application of said nail composition on the clean nail surface,
III. Subsequent application of a new layer of said base coat composition, and
IV. Subsequent application of a new layer of nail polish on the nail.

A method for increasing TOWL is disclosed, wherein said method comprises daily application of the kit of parts to the nails of a subject, and wherein said method comprises the following steps:
I. Removal of any nail polish from the nail using said base coat composition, providing a clean nail surface,
II. Subsequent application of said nail composition on the clean nail surface,
III. Subsequent application of a new layer of said base coat composition, and
IV. Subsequent application of a new layer of nail polish on the nail.

Said TOWL has previously been induced by the application of acetone or nail polish remover to the nail.

Said subsequent application of a new layer of nail polish provides a water-resistant sealing of the nail.

A method for reducing wash-out of a nail composition applied to the nails of a subject is disclosed, wherein said wash-out is due to hand wash, showering and/or increase in relative humidity of the surrounding environment, wherein said method comprises daily application of a kit of parts comprising a base coat composition and a nail composition to the nails of a subject, and wherein said method comprises the following steps:
I. Removal of any nail polish from the nail using said base coat composition, providing a clean nail surface,
II. Subsequent application of said nail composition on the clean nail surface,
III. Subsequent application of a new layer of said base coat composition, and
IV. Subsequent application of a new layer of nail polish on the nail.

A method for preventing filing-induced TOWL is disclosed, wherein said method comprises daily application of a kit of parts comprising a base coat composition and a nail composition to the nails of a subject, and wherein said method comprises the following steps:
I. Removal of any nail polish from the nail using said base coat composition, providing a clean nail surface,
II. Subsequent application of said nail composition on the clean nail surface,
III. Subsequent application of a new layer of said base coat composition, and
IV. Subsequent application of a new layer of nail polish on the nail.

A method for reducing wash-out of a nail composition applied to the nails of a subject is disclosed, wherein said method does not involve any need for applying and/or using additional components.and does not harm and/or degrade and/or destroy the nail.

A method for preventing filing-induced TOWL is disclosed, wherein said method does not involve any need for applying and/or using additional components, and does not harm and/or degrade and/or destroy the nail.

According to an embodiment, the present invention concerns a composition for use in a treatment comprising topical application to nail to be treated for nail fungus or onychomycosis, wherein said composition comprises
a. Water,
b. An alcohol, preferably ethanol,
c. Polyvinyl alcohol,
d. Lactic acid, and/or
e. At least one C1-C4 alkyl ester of lactic acid,malic acid, tartaric acid, or a mixture of any of these.

According to an embodiment, the present invention concerns a composition,wherein said composition further comprises dimethyl isosorbide.

According to an embodiment, the present invention concerns a method for treating nail fungus, wherein said method comprises daily application of said composition to the nails of a subject, and wherein said method comprises the following steps:
I. Removal of any nail polish from the nail using said composition, providing a clean nail surface,
II. Subsequent application of said composition on the clean nail surface, and
III. Subsequent application of a new layer of nail polish on the nail.

Unless otherwise mentioned, all percentages are in weight/weight. Unless otherwise mentioned, all measurements are conducted under standard conditions (ambient temperature and pressure).

### Examples

### Example 1

A kit of parts comprising a base coat composition and a nail composition was provided.

A base coat composition comprising water (61%), alcohol (25%), polyvinyl alcohol (13%), and phenoxyethanol (1%) was provided by mixing of the ingredients.

A nail composition comprising Ethyl Lactate (83%), oil (10%), Lactic acid (1.5%), Caprylic acid (1%), water (3%), Vitamin E (0.5%), polysorbate 20 (0.5%), ascorbyl palmitate (0.4%), and organum vulgare leaf extract (0.1%) was provided by mixing of the ingredients.

### Example 2

A kit of parts comprising a base coat composition and a nail composition was provided.

A base coat composition comprising water (61%), alcohol (25%), polyvinyl alcohol (13%), and phenoxyethanol (1%) was provided by mixing of the ingredients.

A nail composition comprising Ethyl Lactate (87,5%), oil (10%), Lactic acid (1.5%), Caprylic acid (0,9%) and Organum vulgare leaf extract (0,1%) was provided by mixing the ingredients.

### Example 3

A kit of parts comprising a base coat composition and a nail composition was provided.

A base coat composition comprising water (61%), alcohol (25%), polyvinyl alcohol (13%), and phenoxyethanol (1%) was provided by mixing of the ingredients.

A nail composition comprising Ethyl lactate (85.5%), oil (9.8%), Ascorbyl Tetraisopalmitate (2%), Lactic acid (1.5%), Caprylic acid (0.9%), Origanum vulgare Leaf extract (0.1%), Tocopheryl acetate (0.1%), L-Ascorbyl Palmitate (0.1%) was provided by mixing of the ingredients.

### Example 4

A kit of parts of Example 1-3 may be used for the topical treatment of nail fungus.

### Example 5

A kit of parts of Example 1-3 may be applied on nails and/or the skin to prevent and/or treat nail fungus and/or Athlete's foot and/or fungal skin infections.

### Example 6

A kit of parts of Example 1-3 may be applied on nails and/or the skin affected by nail fungus and/or Athlete's foot and/or fungal skin infections to normalize TOWL and/or TEWL in the nail and/or in the skin.

### Example 7

A kit of parts of Example 1-3 may be applied on nails and/or the skin, wherein the base coat hydrates and establishes a low pH under the coat, in which fungus and/or bacteria are inhibited.

### Example 8

A kit of parts of Example 1-3 may be applied on nails and/or the skin, where the base coat establishes a micro-environment, causing the water content and the hydrolysis inside the keratinized areas to increase synergistically.

## Claims

1. A kit of parts comprising a base coat composition and a nail composition,
wherein said base coat composition comprises:
A. Water,
B. An alcohol, preferably ethanol, and
C. Polyvinyl alcohol,
and wherein said nail composition comprises:
a. Lactic acid, and
b. At least one C1-C4 alkyl ester of lactic acid, malic acid, tartaric acid, citric acid, or a mixture of any of these.

2. The kit of parts according to claim 1, wherein said base coat composition is a peel off base coat.

3. The kit of parts according to any of the preceding claims, wherein said base coat composition dries immediately and/or wherein said base coat composition dries within less than 15 minutes, preferably less than 10 minutes, more preferred less than 5 minutes, preferably less than 4 minutes, more preferred less than 3 minutes, preferably less than 2 minutes, more preferred less than 1 minute, preferably less than 30 seconds.

4. The kit of parts according to any of the preceding claims, wherein said base coat composition is free of polyvinyl acetate.

5. The kit of parts according to any of the preceding claims, wherein the total amount (w/w) of said alcohol in said base coat composition is 1 - 90%, preferably 2 - 80%, more preferred 3 - 70%, preferably 4 - 60%, more preferred 5 - 50%, preferably 5 - 40%, more preferred 5 - 35%, preferably 5 - 30%, more preferred 6 - 30%, preferably 7 - 30%, more preferred 8 - 30%, preferably about 8 - 25%.

6. The kit of parts according to any of the preceding claims,
- wherein said alcohol in said base coat composition is selected from the group consisting of a primary alcohol, a secondary alcohol, and a tertiary alcohol and/or wherein said alcohol in said base coat composition is selected from the group consisting of monohydric alcohols, polyhydric alcohols, unsaturated aliphatic alcohols, and alicyclic alcohols, and/or
- wherein said alcohol in said base coat composition is selected from the group consisting of methanol, ethanol, propan-2-ol, butan-1-ol, pentan-1-ol, hexadecan-1-ol, ethane-1,2-diol, propane-1,2-diol, propane-1,2,3-triol, butane-1,2,3,4-tetraol, pentane-1,2,3,4,5-pentol, hexane-1,2,3,4,5,6-hexol, heptane-1,2,3,4,5,6,7-heptol, Prop-2-ene-1-ol, 3,7-Dimethylocta-2,6-dien-1-ol, Prop-2-yn-1-ol, cyclohexane-1,2,3,4,5,6-hexol, and 2-(2-propyl)-5-methyl-cyclohexane-1-ol.

7. The kit of parts according to any of the preceding claims,
- wherein the total amount (w/w) of said polyvinyl alcohol in said base coat composition is 1 - 90%, preferably 2 - 80%, more preferred 3 - 70%, preferably 4 - 60%, more preferred 5 - 50%, preferably 10 - 40%, more preferred 10 - 35%, preferably 15 - 35%, more preferred 15 - 30%, preferably about 15 - 20%, and/or
- wherein said polyvinyl alcohol in said base coat composition is selected from the group consisting of fully saponified grades, partially saponified grades, defoamed grades, fine powder grades, low ash grades, and specialty grades, or a mixture of any of these, and/or
- wherein said polyvinyl alcohol in said base coat composition has a degree of hydrolysis between 65 - 100 mol%, preferably 65 - 99 mol%, more preferred 70 - 99 mol%, preferably 72 - 98 mol%, more preferred 74 - 97 mol%, preferably 76 - 96 mol%, more preferred 78 - 95 mol%, preferably 80 - 95 mol%, more preferred 82 - 94 mol%, preferably 84 - 93 mol%, more preferred 86 - 92 mol%, and/or
- wherein said polyvinyl alcohol in said base coat composition has a pH between 4 - 8, preferably 4.5 - 8, more preferred 4.5 - 7.5, preferably 5 - 7.5, more preferred 5 - 7.

8. The kit of parts according to any of the preceding claims, wherein said base coat composition further comprises a preservative, and/or wherein said preservative is selected from the group consisting of parabens, formaldahyde releasers, isothiazolinones, phenoxyethanol, and organic acids.

9. The kit of parts according to any of the preceding claims, wherein said at least one C1-C4 alkyl ester comprised in said nail composition is ethyl lactate, and/or wherein the total amount (w/w) of C1-C4 alkyl ester, such as ethyl lactate, in said nail composition is 0.05 - 99.9%, preferably 1- 99.5%, more preferred 10 - 99%, preferably 20 - 98%, more preferred 30 - 97%, preferably 40 - 96%, more preferred 50 - 95%, preferably 60 - 94%, more preferred 70 - 93%, preferably 75 - 92%, more preferred 80 - 90%, preferably 80 - 91 %, more preferred 81 - 89%, preferably 82.5 - 87.5%.

10. The kit of parts according to any of the preceding claims, wherein the total amount (w/w) of lactic acid in said nail composition is 0.05 - 40%, preferably 0.1 - 25%, more preferred 0.2 - 15%, preferably 0.4 - 10%, more preferred 0.6 -5%, preferably 0.8 - 3%, more preferred 1 - 2.5%, preferably 1.2 -2%, more preferred about 1.5%.

11. The kit of parts according to any of the preceding claims, wherein said nail
composition further comprises dimethyl isosorbide, wherein the total amount (w/w) of dimethyl isosorbide in said nail composition is 0.005-99.9%, preferably 1-99.5%, more preferred 5-90%, preferably 10-80%, more preferred 15-70%, preferably 20-60%, more preferred 25-50%, preferably 30-40%.

12. The kit of parts according to any of the preceding claims, wherein said nail
composition further comprises an oil, wherein a total amount (w/w) of said oil is between 1 - 90%, preferably 2 - 80%, more preferred 3 - 70%, preferably 4 - 60%, more preferred 5 - 50%, preferably 6 - 40%, more preferred 7 - 30%, preferably 8 - 20%, more preferred 9 - 15%, preferably about 10%, and/or wherein said oil in said nail composition is a plant oil, selected among the group consisting of the plant oils of Flax (Linseed), Hemp (C. sativa), Evening primrose, Safflower, Chia, Kukui (candle nut), Perilla, Grape seed, Pumpkin seed, Sunflower, Walnut, Soybean, Corn, Wheat germ, Rape (Canola), Sesame, Cotton seed, Rice bran, Beechnut, Sweet almond, Olive, Avocado, and mixtures of any of these, or wherein said oil in said nail composition is selected among the group consisting of the plant oils of Hemp Oil, Canola (Rape) Oil, Sunflower Oil, Olive Oil, Avocado Oil, and mixtures of any of these, or wherein said oil in said nail composition is selected among the group consisting of Flax (Linseed) Oil, Hemp Oil, Olive Oil, Sunflower Oil, and mixtures of any of these.

13. The kit of parts according to any of the preceding claims, wherein said base coat composition and/or said nail composition are selected among liquid, a paste, a solid, a cream, an ointment, a nail lacquer, a spray, an oil, a gel, a nail-polish, a serum, an applicator pen, a brush, and a cotton swab.

14. The kit of parts according to any of the preceding claims for use in topical treatment of a nail for nail fungus, and/or for use in treatment of a nail for onychomycosis.

15. The kit of parts for use according to claim 14 for use in application of nail polish on the nails of a subject being treated for nail fungus by using a base coat composition and a nail
composition as defined in any of the preceding claims 1-14, by
I. Removal of any nail polish from the nail using said composition, providing a clean nail surface,
II. Subsequent application of said composition on the clean nail surface, and
III. Subsequent application of a new layer of nail polish on the nail.

## Patentansprüche

1. Teilesatz, umfassend eine Basisbeschichtungszusammensetzung und eine Nagelzusammensetzung,
wobei die Basisbeschichtungszusammensetzung umfasst:
A. Wasser,
B. einen Alkohol, vorzugsweise Ethanol, und
C. Polyvinylalkohol,
und wobei die Nagelzusammensetzung umfasst:
a. Milchsäure, und
b. mindestens einen C1-C4-Alkylester von Milchsäure, Äpfelsäure, Weinsäure, Zitronensäure oder einer Mischung von beliebigen von diesen.

2. Teilesatz nach Anspruch 1, wobei die Basisbeschichtungszusammensetzung eine Abziehbasisbeschichtung ist.

3. Teilesatz nach einem der vorstehenden Ansprüche, wobei die Basisbeschichtungszusammensetzung sofort trocknet und/oder wobei die Basisbeschichtungszusammensetzung innerhalb von weniger als 15 Minuten, vorzugsweise weniger als 10 Minuten, bevorzugter weniger als 5 Minuten, vorzugsweise weniger als 4 Minuten, bevorzugter weniger als 3 Minuten, vorzugsweise weniger als 2 Minuten, bevorzugter weniger als 1 Minute, vorzugsweise weniger als 30 Sekunden trocknet.

4. Teilesatz nach einem der vorstehenden Ansprüche, wobei die Basisbeschichtungszusammensetzung frei von Polyvinylacetat ist.

5. Teilesatz nach einem der vorstehenden Ansprüche, wobei die Gesamtmenge (w/w) des Alkohols in der Basisbeschichtungszusammensetzung 1-90 %, vorzugsweise 2-80 %, bevorzugter 3-70 %, vorzugsweise 4-60 %, bevorzugter 5-50 %, vorzugsweise 5-40 %, bevorzugter 5-35 %, vorzugsweise 5-30 %, bevorzugter 6-30 %, vorzugsweise 7-30 %, bevorzugter 8-30 %, vorzugsweise etwa 8-25 % beträgt.

6. Teilesatz nach einem der vorstehenden Ansprüche,
- wobei der Alkohol in der Basisbeschichtungszusammensetzung ausgewählt ist aus der Gruppe bestehend aus einem primären Alkohol, einem sekundären Alkohol und einem tertiären Alkohol und/oder wobei der Alkohol in der Basisbeschichtungszusammensetzung ausgewählt ist aus der Gruppe bestehend aus einwertigen Alkoholen, mehrwertigen Alkoholen, ungesättigten aliphatischen Alkoholen und alicyclischen Alkoholen und/oder
- wobei der Alkohol in der Basisbeschichtungszusammensetzung ausgewählt ist aus der Gruppe bestehend aus Methanol, Ethanol, Propan-2-ol, Butan-1-ol, Pentan-1-ol, Hexadecan-1-ol, Ethan-1,2-diol, Propan-1,2-diol, Propan-1,2,3-triol, Butan-1,2,3,4-tetraol, Pentan-1,2,3,4,5-pentol, Hexan-1,2,3,4,5,6-hexol, Heptan-1,2,3,4,5,6,7-heptol, Prop-2-en-1-ol, 3,7-Dimethylocta-2,6-dien-1-ol, Prop-2-in-1-ol, Cyclohexan-1,2,3,4,5,6-hexol und 2-(2-Propyl)-5-methylcyclohexan-1-ol.

7. Teilesatz nach einem der vorstehenden Ansprüche,
- wobei die Gesamtmenge (w/w) des Polyvinylalkohols in der Basisbeschichtungszusammensetzung 1 - 90 %, vorzugsweise 2 - 80 %, bevorzugter 3
- 70 %, vorzugsweise 4 - 60 %, bevorzugter 5 - 50 %, vorzugsweise 10 - 40 %, bevorzugter 10 - 35 %, vorzugsweise 15 - 35 %, bevorzugter 15 - 30 %, vorzugsweise etwa 15 - 20 % beträgt und/oder
- wobei der Polyvinylalkohol in der Basisbeschichtungszusammensetzung ausgewählt ist aus der Gruppe bestehend aus vollständig verseiften Qualitäten, teilweise verseiften Qualitäten, entschäumten Qualitäten, feinen Pulverqualitäten, aschearmen Qualitäten und Spezialqualitäten oder einer Mischung von beliebigen von diesen und/oder
- wobei der Polyvinylalkohol in der Basisbeschichtungszusammensetzung einen Hydrolysegrad zwischen 65 - 100 mol%, vorzugsweise 65 - 99 mol%, bevorzugter 70 - 99 mol%, vorzugsweise 72 - 98 mol%, bevorzugter 74 - 97 mol%, vorzugsweise 76 - 96 mol%, bevorzugter 78 - 95 mol%, vorzugsweise 80 - 95 mol%, bevorzugter 82 - 94 mol%, vorzugsweise 84 - 93 mol%, bevorzugter 86 - 92 mol% aufweist und/oder
- wobei der Polyvinylalkohol in der Basisbeschichtungszusammensetzung einen pH-Wert zwischen 4 - 8, vorzugsweise 4,5 - 8, bevorzugter 4,5 - 7,5, vorzugsweise 5 - 7,5, bevorzugter 5 - 7 aufweist.

8. Teilesatz nach einem der vorstehenden Ansprüche, wobei die Basisbeschichtungszusammensetzung ferner ein Konservierungsmittel umfasst und/oder wobei das Konservierungsmittel ausgewählt ist aus der Gruppe bestehend aus Parabenen, Formaldahydabspaltern, Isothiazolinonen, Phenoxyethanol und organischen Säuren.

9. Teilesatz nach einem der vorstehenden Ansprüche, wobei der mindestens eine C1-C4-Alkylester, der in der Nagelzusammensetzung enthalten ist, Ethyllactat ist und/oder wobei die Gesamtmenge (w/w) des C1-C4-Alkylesters, wie etwa Ethyllactat, in der Nagelzusammensetzung 0,05-99,9 %, vorzugsweise 1-99,5 %, bevorzugter 10-99 %, vorzugsweise 20-98 %, bevorzugter 30-97 %, vorzugsweise 40-96 %, bevorzugter 50-95 %, vorzugsweise 60-94 %, bevorzugter 70-93 %, vorzugsweise 75-92 %, bevorzugter 80-90 %, vorzugsweise 80-91 %, bevorzugter 81-89 %, vorzugsweise 82,5-87,5 % beträgt.

10. Teilesatz nach einem der vorstehenden Ansprüche, wobei die Gesamtmenge (w/w) der Milchsäure in der Nagelzusammensetzung 0,05-40 %, vorzugsweise 0,1-25 %, bevorzugter 0,2-15 %, vorzugsweise 0,4-10 %, bevorzugter 0,6-5 %, vorzugsweise 0,8-3 %, bevorzugter 1-2,5 %, vorzugsweise 1,2-2 %, bevorzugter etwa 1,5 % beträgt.

11. Teilesatz nach einem der vorstehenden Ansprüche, wobei die Nagelzusammensetzung ferner Dimethylisosorbid umfasst, wobei die Gesamtmenge (w/w) des Dimethylisosorbids in der Nagelzusammensetzung 0,005-99,9 %, vorzugsweise 1-99,5 %, bevorzugter 5-90 %, vorzugsweise 10-80 %, bevorzugter 15-70 %, vorzugsweise 20-60 %, bevorzugter 25-50 %, vorzugsweise 30-40 % beträgt.

12. Teilesatz nach einem der vorstehenden Ansprüche, wobei die Nagelzusammensetzung ferner ein Öl umfasst, wobei eine Gesamtmenge (w/w) des Öls zwischen 1-90 %, vorzugsweise 2-80 %, bevorzugter 3-70 %, vorzugsweise 4-60 %, bevorzugter 5-50 %, vorzugsweise 6-40 %, bevorzugter 7-30 %, vorzugsweise 8-20 %, bevorzugter 9-15 %, vorzugsweise etwa 10 % beträgt, und/oder wobei das Öl in der Nagelzusammensetzung ein Pflanzenöl ist, ausgewählt aus der Gruppe bestehend aus den Pflanzenölen von Flachs (Leinsamen), Hanf (C. sativa), Nachtkerze, Färberdistel, Chia, Kukui (Kerzennuss), Perilla, Traubenkern, Kürbiskern, Sonnenblume, Walnuss, Sojabohne, Mais, Weizenkeim, Raps (Canola), Sesam, Baumwollsamen, Reiskleie, Bienennuss, Süßmandel, Olive, Avocado und Mischungen von beliebigen davon, oder wobei das Öl in der Nagelzusammensetzung ausgewählt ist aus der Gruppe bestehend aus den Pflanzenölen von Hanföl, Canola (Raps) Öl, Sonnenblumenöl, Olivenöl, Avocadoöl und Mischungen von beliebigen davon, oder wobei das Öl in der Nagelzusammensetzung ausgewählt ist aus der Gruppe bestehend aus Flachs (Leinsamen) Öl, Hanföl, Olivenöl, Sonnenblumenöl und Mischungen von beliebigen davon.

13. Teilesatz nach einem der vorstehenden Ansprüche, wobei die Basisbeschichtungszusammensetzung und/oder die Nagelzusammensetzung ausgewählt sind aus einer Flüssigkeit, einer Paste, einem Feststoff, einer Creme, einer Salbe, einem Nagellack, einem Spray, einem Öl, einem Gel, einem Nagellack, einem Serum, einem Applikatorstift, einem Pinsel und einem Baumwolltupfer.

14. Teilesatz nach einem der vorstehenden Ansprüche zur Verwendung bei der topischen Behandlung eines Nagels gegen Nagelpilz und/oder zur Verwendung bei der Behandlung eines Nagels gegen Onychomykose.

15. Teilesatz zur Verwendung nach Anspruch 14 zur Verwendung bei der Anwendung von Nagellack auf die Nägel eines Subjekts, das gegen Nagelpilz behandelt wird, unter Verwendung einer Basisbeschichtungszusammensetzung und einer Nagelzusammensetzung, wie in einem der vorstehenden Ansprüche 1-14 definiert, durch
I. Entfernen von Nagellack vom Nagel unter Verwendung der Zusammensetzung, wodurch eine saubere Nageloberfläche bereitgestellt wird,
II. anschließendes Auftragen der Zusammensetzung auf die saubere Nageloberfläche und
III. anschließendes Auftragen einer neuen Schicht von Nagellack auf den Nagel.

## Revendications

1. Un kit de pièces comprenant une composition de couche de base et une composition pour les ongles, dans lequel ladite composition de base comprend :
A. de l'eau,
B. un alcool, de préférence l'éthanol, et
C. de l'alcool polyvinylique,
et dans lequel ladite composition pour les ongles comprend :
a. de l'acide lactique, et
b. au moins un ester d'alkyle en C1-C4 de l'acide lactique, de l'acide malique, de l'acide tartrique, de l'acide citrique ou d'un mélange de ceux- ci.

2. Le kit de pièces selon la revendication 1, dans lequel ladite composition de couche de base est une couche de base pelable.

3. Le kit de pièces selon l'une quelconque des revendications précédentes, dans lequel ladite composition de couche de base sèche immédiatement et/ou dans lequel ladite composition de couche de base sèche en moins de 15 minutes, de préférence en moins de 10 minutes, de préférence en moins de 5 minutes, de préférence en moins de 4 minutes, de préférence en moins de 3 minutes, de préférence en moins de 2 minutes, de préférence en moins de 1 minute, de préférence en moins de 30 secondes.

4. Le kit de pièces selon l'une quelconque des revendications précédentes, dans lequel ladite composition de couche de base ne contient pas d'acétate de polyvinyle.

5. Le kit de pièces selon l'une quelconque des revendications précédentes, dans lequel la quantité totale (en poids) dudit alcool dans ladite composition de couche de base est de 1 - 90%, de préférence 2 - 80% et plus préférablement encore 3 - 70%, de préférence 4 - 60% et plus préférablement encore 5 - 50%, de préférence 5 - 40% et plus préférablement encore 5 - 35%, de préférence 5 - 30% et plus préférablement encore 6 - 30%, de préférence 7 - 30% et plus préférablement encore 8 - 30%, de préférence environ 8 - 25%.

6. Le kit de pièces selon l'une quelconque des revendications précédentes,
- dans lequel ledit alcool dans ladite composition de couche de base est choisi dans le groupe constitué par un alcool primaire, un alcool secondaire et un alcool tertiaire et/ou
- dans lequel ledit alcool dans ladite composition de couche de base est choisi dans le groupe constitué d'alcools monohydriques, d'alcools polyhydriques, d'alcools aliphatiques insaturés et d'alcools alicycliques, et/ou
- dans lequel ledit alcool dans ladite composition de couche de base est choisi dans le groupe constitué par le méthanol, l'éthanol, le propan-2-ol, le butan-1-ol, le pentan-1-ol, l'hexadécan-1-ol, l'éthane-1,2-diol, le propane-1,2-diol, le propane-1,2,3-triol, le butane-1,2,3,4-tétraol, le pentane-1,2,3,4,5-pentol, l'hexane-1,2,3,4,5,6-hexol, l'heptane-1,2,3,4,5,6,7-heptol, le Prop-2-ène-1-ol, le 3,7-Diméthylocta-2,6-diène-1-ol, le Prop-2-yn-1-ol, le cyclohexane-1,2,3,4,5,6-hexol et le 2-(2-propyl)-5-méthyl-cyclohexane-1-ol.

7. Le kit de pièces selon l'une quelconque des revendications précédentes,
- dans lequel la quantité totale (en poids) dudit alcool polyvinylique dans ladite composition de couche de base est de 1 - 90%, de préférence 2 - 80%, et plus préférablement encore 3 - 70%, de préférence 4 - 60%, et plus préférablement encore 5 - 50%, de préférence 10 - 40%, et plus préférablement encore 10 - 35%, de préférence 15 - 35%, et plus préférablement encore 15 - 30%, de préférence environ 15 - 20%, et/ou
- dans lequel ledit alcool polyvinylique dans ladite composition de couche de base est choisi dans le groupe constitué par les grades entièrement saponifiés, les grades partiellement saponifiés, les grades démoussés, les grades en poudre fine, les grades à faible teneur en cendres et les grades de spécialité, ou un mélange de l'un de ces grades, et/ou
- dans lequel ledit alcool polyvinylique dans ladite composition de couche de base a un degré d'hydrolyse entre 65 - 100 mol%, de préférence 65 - 99 mol% et plus préférablement encore 70 - 99 mol%, de préférence 72 - 98 mol% et plus préférablement encore 74 - 97 mol%, de préférence 76 - 96 mol% et plus préférablement encore 78 - 95 mol%, de préférence 80 - 95 mol% et plus préférablement encore 82 - 94 mol%, de préférence 84 - 93 mol% et plus préférablement encore 86 - 92 mol%, et / ou
- dans lequel ledit alcool polyvinylique dans ladite composition de couche de base a un pH compris entre 4 et 8, de préférence entre 4,5 et 8 et plus préférablement encore entre 4,5 et 7,5, de préférence entre 5 et 7,5 et plus préférablement encore entre 5 et 7.

8. Le kit de pièces selon l'une quelconque des revendications précédentes, dans lequel ladite composition de couche de base comprend en outre un agent conservateur, et/ou dans lequel ledit agent conservateur est choisi dans le groupe constitué par les parabènes, les libérateurs de formaldéhyde, les isothiazolinones, le phénoxyéthanol et les acides organiques.

9. Le kit de pièces selon l'une quelconque des revendications précédentes, dans lequel ledit au moins un ester d'alkyle en C1-C4 compris dans ladite composition pour les ongles est le lactate d'éthyle, et/ou dans lequel la quantité totale (en poids) d'ester d'alkyle en C1-C4, tel que le lactate d'éthyle, dans ladite composition pour les ongles est de 0,05 - 99,9%, de préférence 1 - 99,5% et plus préférablement encore 10 - 99%, de préférence 20 - 98% et plus préférablement encore 30 - 97%, de préférence 40 - 96% et plus préférablement encore 50 - 95%, de préférence 60 - 94% et plus préférablement encore 70 - 93%, de préférence 75 - 92% et plus préférablement encore 80 - 90%, de préférence 80 - 91% et plus préférablement encore 81 - 89%, de préférence 82,5 - 87,5%.

10. Le kit de pièces selon l'une quelconque des revendications précédentes, dans lequel la quantité totale (en poids) d'acide lactique dans ladite composition pour les ongles est de 0,05 - 40%, de préférence 0,1 - 25% et plus préférablement encore 0,2 - 15%, de préférence 0,4 - 10% et plus préférablement encore 0,6 - 5%, de préférence 0,8 - 3% et plus préférablement encore 1 - 2,5%, de préférence 1,2 - 2% et plus préférablement encore environ 1,5%.

11. Le kit de pièces selon l'une quelconque des revendications précédentes, dans lequel ladite composition pour les ongles comprend en outre de l'isosorbide de diméthyle, dans lequel la quantité totale (en poids) d'isosorbide de diméthyle dans ladite composition pour les ongles est de 0,005-99,9%, de préférence 1-99,5% et plus préférablement encore 5-90%, de préférence 10-80% et plus préférablement encore 15-70%, de préférence 20-60% et plus préférablement encore 25-50%, de préférence 30-40%.

12. Le kit de pièces selon l'une quelconque des revendications précédentes, dans lequel ladite composition pour les ongles comprend en outre une huile, dans lequel une quantité totale (en poids) de ladite huile est comprise entre 1 - 90%, de préférence 2 - 80% et plus préférablement encore 3 - 70%, de préférence 4 - 60% et plus préférablement encore 5 - 50%, de préférence 6 - 40% et plus préférablement encore 7 - 30%, de préférence 8 - 20% et plus préférablement encore 9 - 15%, de préférence environ 10%, et/ou dans lequel l'huile contenue dans ladite composition pour les ongles est une huile végétale choisie dans le groupe constitué par les huiles végétales de lin (graine de lin), chanvre (C. sativa), onagre, carthame, chia, kukui (noix de chandelle), périlla, pépins de raisin, pépins de citrouille, tournesol, noix, soja, maïs, germe de blé, colza (canola), sésame, graine de coton, son de riz, faînes, amandes douces, olives, avocats et mélanges de ces huiles, ou dans lequel ladite huile contenue dans ladite composition pour ongles est choisie dans le groupe constitué par les huiles végétales de chanvre, de canole (colza), de tournesol, d'olive, d'avocat et mélanges de ces huiles, ou dans lequel ladite huile contenue dans ladite composition pour les ongles est choisie dans le groupe constitué par l'huile de lin, l'huile de chanvre, l'huile d'olive, l'huile de tournesol et des mélanges de ces huiles.

13. Le kit de pièces selon l'une quelconque des revendications précédentes, dans lequel ladite composition de couche de base et/ou ladite composition pour les ongles sont choisies parmi un liquide, une pâte, un solide, une crème, une pommade, un vernis à ongles, un spray, une huile, un gel, un vernis à ongles, un sérum, un stylo applicateur, un pinceau et un coton-tige.

14. Le kit de pièces selon l'une quelconque des revendications précédentes destiné à être utilisé pour le traitement topique d'un ongle contre la mycose des ongles, et/ou destiné à être utilisé pour le traitement d'un ongle contre l'onychomycose.

15. Le kit de pièces destiné à être utilisé selon la revendication 14 pour l'application d'un vernis à ongles sur les ongles d'un patient traité pour une mycose des ongles en utilisant une composition de couche de base et une composition pour les ongles telle que définies selon l'une quelconque des revendications précédentes 1 à 14, par
I. enlèvement de tout vernis à ongles de l'ongle en utilisant ladite composition, ce qui permet d'obtenir une surface propre de l'ongle,
II. application ultérieure de ladite composition sur la surface propre de l'ongle, et
III. application ultérieure d'une nouvelle couche de vernis sur l'ongle.
